# EUROPÄISCHE PATENTSCHRIFT

(11) **EP 1 568 362 B1**
(45) Veröffentlichungstag und Bekanntmachung des Hinweises auf die Patenterteilung: **27.07.2011**
(21) Anmeldenummer: 05003504.7
(22) Anmeldetag: 18.02.2005
(51) Int. Cl.: A61K 9/20

(54) **Viertelbare Tablette**
Divisible tablet
Comprimé sécable

(30) Priorität: 20.02.2004 DE 202004002700 U
(43) Veröffentlichungstag der Anmeldung: 31.08.2005
(73) Patentinhaber: Desitin Arzneimittel GmbH, D-22311 Hamburg (DE)
(72) Erfinder: Lennartz, Peter, Dr., 20253 Hamburg (DE)
(74) Vertreter: UEXKÜLL & STOLBERG

(56) Entgegenhaltungen:
- EP-A- 0 531 964
- US-A- 4 353 887
- US-A- 4 824 677

## Beschreibung

Die vorliegende Erfindung betrifft eine viertelbare Tablette.

Die Teilung von Tabletten ist ein bekanntes und gängiges Verfahren, die Applikation von Teildosierungen zu ermöglichen. Dies wird in der Regel durch Anbringung von Teilkerben auf den Tabletten realisiert. Dies ermöglicht die Teilung der Tabletten entlang der Teilkerben.

In der Europäischen Pharmakopöe 4.01 werden erstmals Anforderungen an die Gewichtseinheitlichkeit von Tablettenteilstücken bei Arzneimitteln gestellt. Die Tablettenteilstücke müssen die gleichen Anforderungen erfüllen wie ungeteilte Tabletten. Diese Anforderungen sind mit üblichen Tablettenformen nur schwer zu erfüllen, insbesondere wenn das Tablettengewicht über 160 mg liegt.

Grundsätzlich sind Tabletten bekannt, die einen Tablettenkörper mit im Wesentlichen runder Grundform und einer Oberseite und einer Unterseite haben, wobei die Unterseite konvex gewölbt ist und die Oberseite im Wesentlichen eben ist und mit zwei rechtwinklig zueinander angeordneten V-förmigen Teilkerben versehen ist. Das Teilungsergebnis einer Tablettenteilung per Hand ist jedoch im hohen Maße vom Durchführenden und der Teilungsmethode abhängig. Dabei haben gerade ältere Patienten häufig Probleme mit der Handhabung.

Verschiedene Wirkstoffe, wie z.B. Antiepilektika, insbesondere Lamotrigin, müssen z.B. auf Grund unerwünschter Nebenwirkungen und langer Halbwertszeit genau und einschleichend dosiert werden. Dabei wird die Dosis über mehrere Wochen langsam erhöht. Dies macht eine Vielzahl unterschiedlicher Dosierungsschritte erforderlich. Ideal für die Aufdosierung sind Dosierungen von 25 mg, 50 mg, 75 mg und 100 mg.

Somit besteht ein Bedarf für eine Tablette, die sich auf einfache Weise in vier Teilstücke mit hoher Gewichtskonstanz entsprechend der Vorgaben teilen lässt.

Es ist Aufgabe der vorliegenden Erfindung solche viertelbaren Tabletten zu schaffen, die sich einfach und mit möglichst geringer Gewichtsvariation der Teilstücke teilen lassen.

Zur Lösung dieser Aufgabe dient die viertelbare Tablette mit den Merkmalen des Anspruchs 1. Vorteilhafte Ausgestaltungen der Erfindung sind in den Unteransprüchen angegeben.

Die erfindungsgemäße Tablette enthält als Wirkstoff vorzugsweise mindestens ein Antiepileptikum, insbesondere Lamotrigin.

Die Tablette hat einen im Wesentlichen quaderförmigen Tablettenkörper, der eine im Wesentlichen quadratische Grundform hat. Der Tablettenkörper hat eine Oberseite und eine Unterseite, wobei die Unterseite konvex vorgewölbt ist und die Oberseite im Wesentlichen eben ist. Auf der Oberseite sind zwei rechtwinklig zueinander angeordnete, im Querschnitt V-förmige Teilkerben vorgesehen, die die quadratische Grundform des Tablettenkörpers in vier gleich große Teilquadrate unterteilen. Die V-förmigen Teilkerben auf der Oberseite gehen an ihren Enden in V-förmige Teilkerben in den zwischen der Oberseite und der Unterseite verlaufenden Seitenwänden des Tablettenkörpers über.

Für die Teilung wird die Tablette mit ihrer konvex gewölbten Unterseite auf eine Unterlage gelegt. Dann wird durch Druck auf die plane Oberseite die Tablette geteilt.

Durch die erfindungsgemäße Form der Tablette und die Anordnung der Teilkerben wird eine hohe und verläßlich reproduzierbare Gewichtskonstanz der Tablettenteile sichergestellt. Dies liegt insbesondere auch an den im Vergleich zu bekannten Tabletten kleinen Bruchflächen, die bei der Teilung der Tablette entstehen und die durch variierende Bruchverläufe wesentlich zum Entstehen von Gewichtsunterschieden bei der Teilung der Tabletten beitragen.

Mit der erfindungsgemäßen Formgebung kann nämlich, für eine vorgegebene Menge von Tablettenmaterial, der Tablettenkörper mit quadratischer Grundform eine kleinere Seitenlänge der quadratischen Grundform haben, als der Durchmesser einer Tablette mit kreisrunder Grundform. Insofern ist die Länge der Bruchfläche unter der V-förmigen Teilkerbe in der Oberseite der Tablette geringer als die Länge der Bruchkante der entsprechenden Tablette mit kreisförmiger oder anderweitiger Grundform.

In einer bevorzugten Ausführungsform kann die quadratische Grundform des quaderförmigen Tablettenkörpers abgerundete Ecken aufweisen.

Die Wölbungshöhe der konvexgeformten Unterseite des Tablettenkörpers beträgt etwa 1,1 bis 1,3 mm, d.h. der Scheitelpunkt der Wölbung liegt etwa 1,1 bis 1,3 mm über einer gedachten planen Fläche, die an die Seitenwände des Tablettenkörpers anschließt.

Der Öffnungswinkel der im Querschnitt V-förmigen Teilkerben der Oberseite liegt vorzugsweise im Bereich zwischen 80° und 100°.

Der Öffnungswinkel der V-förmigen Teilkerben in den Seitenwänden des Tablettenkörpers liegt vorzugsweise im Bereich von 50° bis 120°, insbesondere 100° bis 120° oder 50° bis 70°.

Die Kerbtiefe der V-förmigen Teilkerben auf der Oberseite liegt vorzugsweise im Bereich von 0,9 bis 1,1 mm.

In einer anderen bevorzugten Ausführungsform ist die Kerbtiefe der V-förmigen Teilkerben auf der Oberseite nicht konstant. Vorzugsweise kann die Kerbtiefe der v-förmigen Teilkerben auf der Oberseite der Tablette von den Rändern des Tablettengrundkörpers ausgehend zu dem Schnittpunkt der Teilkerben hin einem Maximum zustreben, d.h. die Kerbtiefe ist im Schnittpunkt der Teilkerben am größten, z.B. 0,9 bis 1,1 mm, und nimmt entlang der Teilkerben zu den Rändern der Tablette hin ab. Des Weiteren kann es bevorzugt sein, dass in einer Seitenansicht der Tablette, die den Querschnitt der Tablette entlang einer V-förmigen Teilkerbe auf der Oberseite zeigt, der Grund der Teilkerbe, d.h. die Linie, welche die tiefsten Punkte einer Kerbe miteinander verbindet, einen im Wesentlichen bogenförmigen und insbesondere kreisförmigen Verlauf aufweist. Vorzugsweise liegt der Radius des Kreises, den der Grund der V-förmigen Teilkerbe auf der Oberseite beschreibt, im Bereich von 5,0 bis 12,0 mm. Vorzugsweise bleibt der Öffnungswinkel der V-förmigen Teilkerben auf der Oberseite bei diesen Ausführungsformen konstant, sodass bei einer Aufsicht auf die Oberseite der Tablette die Breite der V-förmigen Teilkerben auf der Oberseite ausgehend vom Mittelpunkt der Tablette zu den Rändern hin abnimmt.

Die plane Oberseite des Tablettenkörpers hat vorzugsweise in ihren Randbereichen eine Facettierung, d.h. einen abgeschrägten Randbereich. Die Abschrägung liegt vorzugsweise unter einem Winkel von 20° bis 40°, insbesondere 20 bis 30° relativ zu der planen Oberfläche der Oberseite.

Die Seitenlänge der quadratischen Grundform des Tablettenkörpers liegt vorzugsweise im Bereich von 9 bis 11 mm. Somit liegt das Verhältnis der Wölbungshöhe der konvexgeformten Unterseite zu der Seitenlänge der Tablettengrundform vorzugsweise im Bereich von 0,12 bis 0,15 und das Verhältnis der Kerbtiefe der V-förmigen Teilkerben auf der Oberseite in deren Schnittpunkt zu der Seitenlänge der Tablettengrundform vorzugsweise im Bereich von 0,08 bis 0,13.

Weiterhin kann die Unterseite einer erfindungsgemäßen Tablette ebenfalls Teilkerben, insbesondere im Querschnitt V-förmige Teilkerben aufweisen. Vorzugsweise ist die Unterseite mit zwei rechtwinklig zueinander angeordneten im Querschnitt V-förmigen Teilkerben versehen ist, die parallel zu den V-förmigen Teilkerben auf der Oberseite verlaufen. Dabei liegt der Öffnungswinkel der V-förmigen Teilkerben auf der Unterseite des Tablettenkörpers vorzugsweise im Bereich zwischen 80° und 100° und die Kerbtiefe der V-förmigen Teilkerben auf der Unterseite vorzugsweise im Bereich von 0,1 bis 0,4 mm.

Eine bevorzugte Tablette hat ein Gesamtgewicht im Bereich von 300 bis 350 mg. Vorzugsweise enthält die Tablette etwa 100 mg an Wirkstoff, wie Lamotrigin.

Die Erfindung wird im Folgenden anhand eines Ausführungsbeispiels in den Zeichnungen erläutert, in denen:
- Figur 1: eine Draufsicht auf eine erfindungsgemäße Tablette von oben ist,
- Figur 2: eine Seitenansicht der Tablette aus Figur 1 ist,
- Figur 3: eine Aufsicht von unten auf die Tablette aus Figur 1 ist, und
- Figur 4: eine Aufsicht auf die Oberseite einer erfindungsgemäßen Tablette ist, bei der die Breite der V-förmigen Teilkerben auf der Oberseite nicht konstant ist.

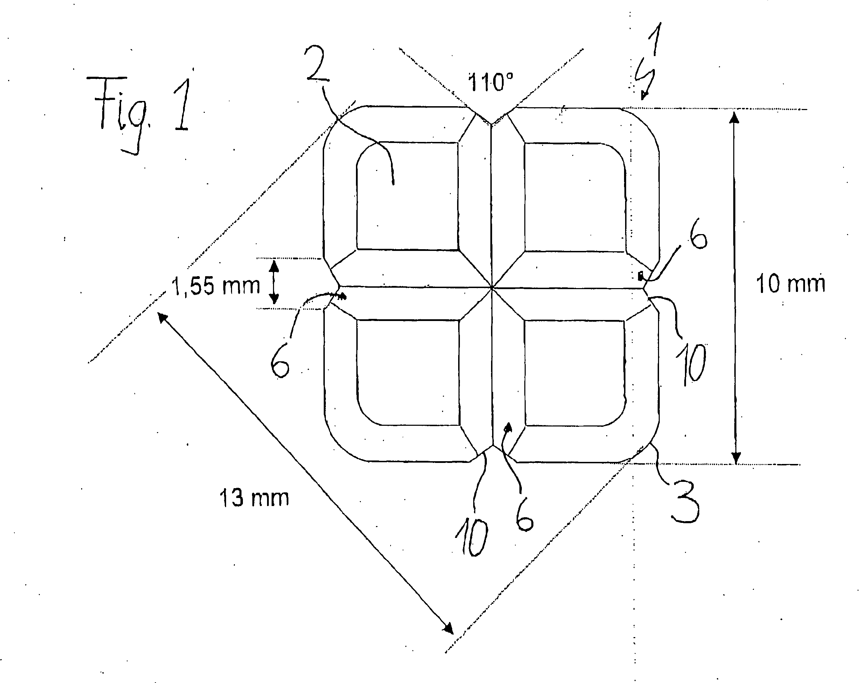

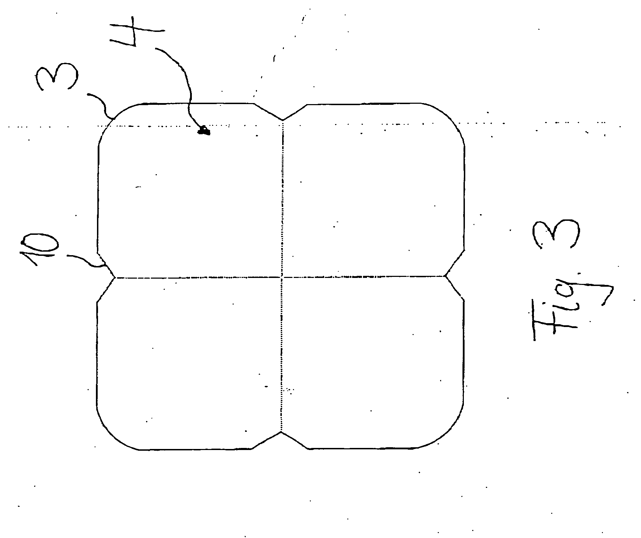

Wie in den Figuren 1 und 3 zu sehen, hat die Tablette einen Tablettenkörper 1 mit einer in der Draufsicht von oben (Figur 1) und von unten (Figur 3) im Wesentlichen quadratischen Grundform, wobei allerdings leicht abgerundete Ecken 3 vorgesehen sind. Die Kantenlänge der quadratischen Grundform beträgt in der dargestellten Ausführungsform z.B. 10 mm.

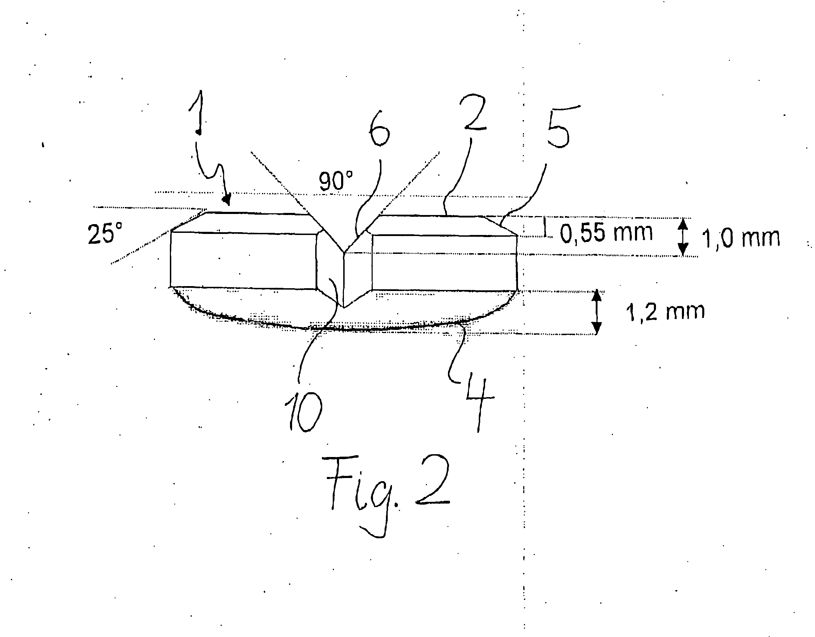

Wie in Figuren 1 und 2 zu erkennen ist, sind auf der Oberseite zwei im Querschnitt zu ihrer Längsrichtung V-förmige Teilkerben 6 vorgesehen, die rechtwinklig zueinander stehen und sich im Mittelpunkt des Tablettenkörpers schneiden, so dass sie den Tablettenkörper 1 in vier gleich große Teilstücke unterteilen.

Die V-förmigen Teilkerben 6 auf der Oberseite 2 gehen an ihren Enden in ebenfalls V-förmige Teilkerben 10 in den Seitenwänden des Tablettenkörpers über, die zwischen der Oberseite 2 und der Oberseite 4 verlaufen.

Die Oberseite 2 ist eben, wobei lediglich im Randbereich 5 eine Abschrägung oder Facettierung vorgesehen ist. Die Unterseite 4 des Facettenkörpers ist mit einer konvexen Wölbung ausgebildet. Durch die konvexe Unterseite 4 kann die Tablette nach Auflage auf eine Unterlage durch Drücken auf ihre gegenüberliegenden Seiten geteilt werden, wobei die Bruchfläche entlang der Ebene verläuft, die durch die Scheitellinien der V-förmigen Teilkerben 6 und 10 definiert ist.

In der dargestellten Ausführungsform haben die V-förmigen Teilkerben 6 in der Oberseite 2 einen Öffnungswinkel von 90° und die V-förmigen Teilkerben 10 in den Seitenwänden einen Öffnungswinkel von 110°. Ferner haben die V-förmigen Teilkerben 6 in der Oberseite 2 gegenüber deren planen Oberfläche eine Tiefe von 1,0 mm. die Breite der Teilkerben 10 in den Seitenwänden an ihren äußeren Enden beträgt etwa 1,55 mm.

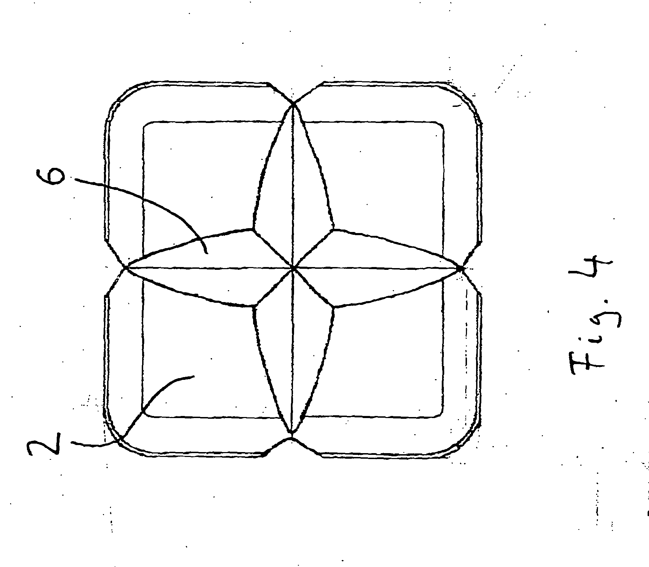

Figur 4 zeigt eine Draufsicht auf eine erfindungsgemäße Tablette von oben, bei der die Breite der V-förmigen Teilkerben 6 auf der Oberseite 2 aufgrund einer zu den Rändern hin abnehmender Kerbtiefe und einem konstanten Öffnungswinkel der Teilkerbe nicht konstant ist.

## Patentansprüche

1. Viertelbare Tablette mit einem Tablettenkörper (1) mit im Wesentlichen quadratischer Grundform und mit einer Oberseite (2) und einer Unterseite (4), wobei die Unterseite (4) konvex gewölbt ist und die Oberseite im Wesentlichen eben und mit zwei rechtwinklig zueinander angeordneten V-förmigen Teilkerben (6) versehen ist, die die quadratische Grundform des Tablettenkörpers in vier gleich große Teilquadrate unterteilen und die an ihren Enden in V-förmige Teilkerben (10) in den zwischen der Oberseite und der Unterseite verlaufenden Seitenwänden des Tablettenkörpers übergehen.

2. Viertelbare Tablette nach Anspruch 1, wobei die im Wesentlichen quadratische Grundform des Tablettenkörpers abgerundete Ecken (3) aufweist.

3. Viertelbare Tablette nach einem der vorhergehenden Ansprüche, wobei die Wölbungshöhe der konvexgeformten Unterseite (4) etwa 1,1 bis 1,3 mm beträgt.

4. Viertelbare Tablette nach einem der vorhergehenden Ansprüche, wobei der Öffnungswinkel der V-förmigen Teilkerben (6) der Oberseite des Tablettenkörpers im Bereich zwischen 80° und 100° liegt.

5. Viertelbare Tablette nach einem der vorhergehenden Ansprüche, wobei der Öffnungswinkel der V-förmigen Teilkerben (10) in den Seitenwänden des Tablettenkörpers im Bereich von 50° bis 120°, insbesondere 100° bis 120° liegt.

6. Viertelbare Tablette nach einem der vorhergehenden Ansprüche, wobei die Kerbtiefe der V-förmigen Teilkerben (6) auf der Oberseite (2) im Bereich von 0,9 bis 1,1 mm liegt.

7. Viertelbare Tablette nach einem der vorhergehenden Ansprüche, wobei die Oberseite (2) des Tablettenkörpers in ihren Randbereichen (5) eine Facettierung aufweist.

8. Viertelbaze Tablette nach Anspruch 7, wobei der Facettenwinkel der Abschrägung in den Randbereichen (5) der Oberseite im Bereich von 20° bis 40°, insbesondere 20° bis 30° liegt.

9. Viertelbare Tablette nach einem der vorhergehenden Ansprüche, wobei die Seitenlänge der quadratischen Grundform des Tablettenkörpers im Bereich von 9 bis 11 mm liegt.

10. Viertelbare Tablette nach einem der vorhergehenden Ansprüche, wobei das Gewicht der Tablette im Bereich von 300 bis 350 mg liegt.

11. Viertelbare Tablette nach einem der vorhergehenden Ansprüche, wobei der Wirkstoff mindestens ein Antiepileptikum ist.

12. Viertelbare Tablette nach einem der vorhergehenden Ansprüche, wobei der Wirkstoff Lamotrigin ist.

13. Viertelbare Tablette nach einem der vorhergehenden Ansprüche, die etwa 100 mg Wirkstoff enthält.

14. Viertelbare Tablette nach einem der vorhergehenden Ansprüche, wobei die Kerbtiefe der V-förmigen Teilkerben (6) auf der Oberseite (2) in ihrem Schnittpunkt am größten ist und entlang der Teilkerben zu den Rändern der Tablette hin abnimmt.

15. Viertelbare Tablette nach einem der vorhergehenden Ansprüche, wobei die Unterseite (4) auch Teilkerben aufweist.

16. Viertelbare Tablette nach Anspruch 15, wobei die Unterseite (4) mit zwei rechtwinklig zueinander angeordneten im Querschnitt V-förmigen Teilkerben versehen ist, die parallel zu den V-förmigen Teilkerben (6) auf der Oberseite (2) verlaufen.

17. Viertelbare Tablette nach Anspruch 15 oder 16, wobei der Öffnungswinkel der V-förmigen Teilkerben auf der Unterseite (4) des Tablettenkörpers im Bereich zwischen 80° und 100° liegt.

18. Viertelbare Tablette nach einem der Ansprüche 15 bis 17, wobei die Kerbtiefe der V-förmigen Teilkerben auf der Unterseite (4) im Bereich von 0,1 bis 0,4 mm liegt.

## Claims

1. Tablet which is divisible into four parts, said tablet having a tablet body (1) with an essentially square basic form and with an upper side (2) and a lower side (4), wherein the lower side (4) is convexly curved and the upper side is essentially planar and is provided with two V-shaped dividing notches (6) arranged at right angles to one another, which divide the square basic form of the tablet body into four equally large square elements and which merge at their ends into V-shaped dividing notches (10) in the side walls of the tablet body that extend between the upper side and the lower side.

2. Tablet which is divisible into four parts according to claim 1, wherein the essentially square basic form of the tablet body has rounded corners (3).

3. Tablet which is divisible into four parts according to any one of the preceding claims, wherein the height of curvature of the convexly shaped lower side (4) is about 1.1 to 1.3 mm.

4. Tablet which is divisible into four parts according to any one of the preceding claims, wherein the aperture angle of the V-shaped dividing notches (6) of the upper side of the tablet body is in the range between 80° and 100°.

5. Tablet which is divisible into four parts according to any one of the preceding claims, wherein the aperture angle of the V-shaped dividing notches (10) in the side walls of the tablet body is in the range of from 50° to 120°, particularly from 100° to 120°.

6. Tablet which is divisible into four parts according to any one of the preceding claims, wherein the notch depth of the V-shaped nicks (6) on the upper side (2) is in the range of from 0.9 to 1.1 mm.

7. Tablet which is divisible into four parts according to any one of the preceding claims, wherein the upper side (2) of the tablet body has a faceting in its marginal areas (5).

8. Tablet which is divisible into four parts according to claim 7, wherein the facet angle of the bevel in the marginal areas (5) of the upper side is in the range of from 20°to 40°, particularly from 20° to 30°.

9. Tablet which is divisible into four parts according to any one of the preceding claims, wherein the side length of the square basic form of the tablet body is in the range of from 9 to 11 mm.

10. Tablet which is divisible into four parts according to any one of the preceding claims, wherein the weight of the tablet is in the range of from 300 to 350 mg.

11. Tablet which is divisible into four parts according to any one of the preceding claims, wherein the active agent is at least one antiepileptic.

12. Tablet which is divisible into four parts according to any one of the preceding claims, wherein the active agent is Lamotrigine.

13. Tablet which is divisible into four parts according to any one of the preceding claims, which contains about 100 mg of active agent.

14. Tablet which is divisible into four parts according to any one of the preceding claims, wherein the notch depth of the V-shaped dividing notches (6) on the upper side (2) is largest at their intersection and decreases along the dividing notches towards the edges of the tablet.

15. Tablet which is divisible into four parts according to any one of the preceding claims, wherein the lower side (4) also has dividing notches.

16. Tablet which is divisible into four parts according to claim 15, wherein the lower side (4) is provided with two dividing notches arranged at right angles to one another, which are V-shaped in their cross-sections and which run parallel to the V-shaped dividing notches (6) on the upper side (2).

17. Tablet which is divisible into four parts according to claim 15 or 16, wherein the aperture angle of the V-shaped dividing notches on the lower side (4) of the tablet body is in the range between 80° and 100°.

18. Tablet which is divisible into four parts according to any one of claims 15 to 17, wherein the notch depth of the V-shaped dividing notches on the lower side (4) is in the range of 0.1 to 0.4 mm.

## Revendications

1. Comprimé quadrisécable comportant un corps de comprimé (1) présentant une forme de base essentiellement carrée et une face supérieure (2) et une face inférieure (4), la face inférieure (4) étant courbée de façon convexe et la face supérieure étant essentiellement plane et étant dotée de deux fentes de fractionnement en forme de V (6) disposées à angle droit l'une par rapport à l'autre, qui divisent la forme de base carrée du corps de comprimé en quatre carrés de même taille et qui, au niveau de leurs extrémités, sont en continuité avec des fentes de fractionnement en forme de V (10) situées dans les parois latérales qui s'étendent entre la face supérieure et la face inférieure du corps de comprimé.

2. Comprimé quadrisécable selon la revendication 1, dans lequel la forme de base essentiellement carrée du corps de comprimé présente des coins arrondis (3).

3. Comprimé quadrisécable selon l'une des revendications précédentes, dans lequel la hauteur de courbure de la face inférieure de forme convexe (4) est compris entre 1,1 et 1,3 mm.

4. Comprimé quadrisécable selon l'une des revendications précédentes, dans lequel l'angle d'ouverture des fentes de fractionnement en forme de V (6) de la face supérieure du corps de comprimé est compris entre 80° et 100°.

5. Comprimé quadrisécable selon l'une des revendications précédentes, dans lequel l'angle d'ouverture des fentes de fractionnement en forme de V (10) dans les parois latérales du corps de comprimé est compris entre 50° et 120°, en particulier entre 100° et 120°.

6. Comprimé quadrisécable selon l'une des revendications précédentes, dans lequel la profondeur de fente des fentes de fractionnement en forme de V (6) sur la face supérieure (2) est comprise entre 0,9 et 1,1 mm.

7. Comprimé quadrisécable selon l'une des revendications précédentes, dans lequel la face supérieure (2) du corps de comprimé présente un biseautage de ses bords extérieurs (5).

8. Comprimé quadrisécable selon la revendication 7, dans lequel l'angle de facette du biseau dans les zones de bordure (5) de la face supérieure est compris entre 20° et 40°, en particulier entre 20° et 30°.

9. Comprimé quadrisécable selon l'une des revendications précédentes, dans lequel la longueur latérale de la forme de base carrée du corps de comprimé est comprise entre 9 et 11 mm.

10. Comprimé quadrisécable selon l'une des revendications précédentes, dans lequel le poids du comprimé est compris entre 300 et 350 mg.

11. Comprimé quadrisécable selon l'une des revendications précédentes, dans lequel la substance active est au moins un anti-épileptique.

12. Comprimé quadrisécable selon l'une des revendications précédentes, dans lequel la substance active est la lamotrigine.

13. Comprimé quadrisécable selon l'une des revendications précédentes qui contient environ 100 mg de substance active.

14. Comprimé quadrisécable selon l'une des revendications précédentes, dans lequel la profondeur de fente de la fente de fractionnement en forme de V (6) sur la face supérieure (2) est la plus importante au niveau du point d'intersection et diminue le long de la fente de fractionnement vers les bords du comprimés.

15. Comprimé quadrisécable selon l'une des revendications précédentes, dans lequel la face inférieure (4) présente également des fentes de fractionnement.

16. Comprimé quadrisécable selon la revendication 15, dans lequel la face inférieure (4) est dotée de deux fentes de fractionnement en forme de V disposées perpendiculairement à angle droit l'une par rapport à l'autre, qui évoluent parallèlement aux fentes de fractionnement en forme de V (6) sur la face supérieure (2).

17. Comprimé quadrisécable selon la revendication 15 ou 16, dans lequel l'angle d'ouverture des fentes de fractionnement en forme de V sur la face inférieure (4) du corps de comprimé est compris entre 80° et 100°.

18. Comprimé quadrisécable selon l'une des revendications 15 à 17, dans lequel la profondeur de fente de la fente de fractionnement en V sur la face inférieure (4) est comprise entre 0,1 et 0,4 mm.
